# Europäisches Patentamt
## European Patent Office
## Office européen des brevets

(19)

(11) Veröffentlichungsnummer: **0 324 858**
**A1**

(12)

# EUROPÄISCHE PATENTANMELDUNG
## Veröffentlicht nach Art. 158 Abs. 3 EPÜ

(21) Anmeldenummer: **88905035.7**

(22) Anmeldetag: **27.01.88**

Daten der zugrundeliegenden internationalen Anmeldung:

(86) Internationale Anmeldenummer:
**PCT/SU88/00020**

(87) Internationale Veröffentlichungsnummer:
**WO89/00406 (26.01.89 89/03)**

(51) Int. Cl.³: **A 61 B 17/11**

(30) Priorität: **14.07.87 SU 4268008**

(43) Veröffentlichungstag der Anmeldung:
**26.07.89 Patentblatt 89/30**

(84) Benannte Vertragsstaaten:
**BE CH DE FR GB IT LI LU NL SE**

(71) Anmelder: **LIPATOV, Viktor Alexeevich**
**ul. Profsojuznaya, 91-3-27**
**Moscow, 117279(SU)**

(71) Anmelder: **GUSKOV, Igor Alexeevich**
**ul. Tulinskaya, 10-1-80**
**Moscow, 109033(SU)**

(71) Anmelder: **KANSHIN, Nikolai Nikolaevich**
**ul. M.Filevskaya, 68-10**
**Moscow, 121433(SU)**

(72) Erfinder: **LIPATOV, Viktor Alexeevich**
**ul. Profsojuznaya, 91-3-27**
**Moscow, 117279(SU)**

(72) Erfinder: **GUSKOV, Igor Alexeevich**
**ul. Tulinskaya, 10-1-80**
**Moscow, 109033(SU)**

(72) Erfinder: **KANSHIN, Nikolai Nikolaevich**
**ul. M.Filevskaya, 68-10**
**Moscow, 121433(SU)**

(74) Vertreter: **Finck, Dieter et al,**
**Patentanwälte v. Füner, Ebbinghaus, Finck Mariahilfplatz**
**2 & 3**
**D-8000 München 90(DE)**

(54) **CHIRURGISCHE NÄHVORRICHTUNG.**

(57) Chirurgisches Nähgerät zum Anlegen von kreisförmigen Kompressions anastomosen an die Organen des Verdauungstraktes enthält eine Stange (2), die im Inneren des Hohlgehäuses (1) untergebracht ist und einen unbeweglichen Verbindungsring (13) trägt, ein hohles zylindrisches Messer (3), das an der Hohlröhre (4), die im Hohlgehäuse (1) koaxial angeordnet ist, befestigt ist, in deren Innerem die Stange (2) hindurchläuft, eine im Hohlgehäuse (1) untergebrachte Nadelvorrichtung (5), die einen ersteren Bohrungsring (6) mit einer Mehrzahl der Bohrungen (8) drin und mit diesem koaxialen anderen Nadelring (7) mit den an diesem befestigten Nadeln (9) trägt, die durch den Verbindungsring (13) beim Anlegen von Anastomosen hundurchstechen. Der Nadelnring (7) stößt gegen einen Stößel (19), der mit einer inneren Abschragung (20) versehen ist und eine mit der Anschlagsflanke (22) im Hohlgehäuse (1) in Eingriff tretende Anschlagsflanke (21) aufweist. Das Nähgerät schließt noch eine auf der Hohlröhre (4) aufgesetzte Schlitzbuchse (23), die mit einem kegeligen Außenbund (24) versehen ist, der mit der inneren Abschrägung (20) des Stößels in Wechselwirkung steht, und einen Feststeller (25) der Schlitzbuchse (23) einschließt, der auf der Hohlröhre (4) angeordnet ist, ein.

./...

EP 0 324 858 A1

FIG.1

0324858

# CHIRURGISCHES NÄHGERÄT

## Technisches Gebiet

Die vorliegende Erfindung bezieht sich auf das Gebiet chirurgische Mechanismen, insbesondere auf chirurgische Nähgeräte.

## Zugrundeliegender Stand der Technik

Bisher ist bekannt ein chirurgisches Nähgerät, das zum Anlegen von Anastomosen an die röhrenförmigen Organe, beispielsweise an die Organe des Verdauungstraktes vermittels der Klammernaht vorausbestimmt ist und eine Matrize, ein zumindest einen Ring aus chirurgischen Klammern tragenden Zylindergehäuse, ein Klammernverstellwerk zum Vorschieben der chirurgischen Klammern und ein zylindrisches Skalpell vorsieht, durch dessen Hilfe die Klammern durch die röhrenförmigen Organe hindurchstechen und auf der Oberfläche der jeweiligen Matrizen zusammengebogen werden, während das Zylinderskalpell das Übergewebe der röhrenförmigen Organe des Organismus abschneidet (GB, A, 2133735).

In diesem bekannten Nähgerät werden die auf lange Dauer bzw. auf die Stetigkeit innerhalb des Organismus des Patienten zurückbleibenden Klammern ausgenutzt. Die Klammern bringen eine luftundichte Klammernnaht zustande, durch welche infolge Operation jeweilige Komplikationen in Kauf genommen werden müssen.

Bekannt ist weiter ein chirurgisches Nähgerät, das zum Anlegen der ringförmigen Kompressionsanastomosen an den Organen des Verdauungskanals dient. Das betreffende Nähgerät enthält eine den ersteren Bohrungsring mit einer Mehrzahl von Bohrungen drin und den anderen Nadelring mit den an diesem befestigten Nadeln aufweisende Nadelvorrichtung, in der die Verteilung der Nadeln mit dieser der Bohrungen in dem ersteren Bohrungsring übereinstimmt und zumindest ein Teil der Nadeln an ihrem freien Ende einen Dichtkegel trägt. Beide Ringe sind innerhalb des Hohlgehäuses einander koaxial untergebracht, in dessen Innerem auch ein Sock ebenfalls koaxial befindlich ist, der an seinem einen Ende einen unbeweglichen Verbindungsring trägt, der vermittels von Rundmutter montiert ist. Bei dem Anlegen der Anastomosen stechen die Nadeln den erwähnten Verbindungsring durch und ein Teil davon in dem betreffenden Verbindungsring durch die Dichtkegel festeingeklemmt wird.

Das betreffende Gerät enthält auch ein zylindrisches Messer, dessen innerer Durchmesser mit dem größten Durchmesser der Rundmutter gleichgroß ist, während das Messer selbst an einer innerhalb des Hohlgehäuses untergebrachten Hohlröhre befestigt ist. In dieser Hohlröhre ist auch der Stock koaxial untergebracht. Das zylindrische Messer ist mit einem Antriebswerk zu dessen Verstellung versehen, das mit der Hohlröhre in Wechselwirkung tritt und einen beweglichen Handgriff aufweist, der in bezug auf das Hohlgehäuse gedreht werden kann und einen in die Schlitze der Hohlröhre eingreifenden Hebel besitzt (PCT/SU, 79/00049).

Die erwähnten Ringe der Nadelvorrichtung und der Verbindungsring sind alle aus elastischen oder plastischen Werkstoffen hergestellt, die folgende Eigenschaften aufweisen. Sie müssen biologisch inert, nichttoxisch, nichtkanzerogen sein.

Zwecks Anlegens einer End-an End-Anastomose muß das Nähgerät innerhalb des röhrenförmigen Organs hineingeschoben werden. Dabei werden die Enden vom Gewebe der Operationsorgane vermittels der Tabaksbeutelnähte an dem Stock befestigt. Nach dem Zusammenführen der Arbeitsteile des Nähgeräts und nach der Annäherung der Nadelvorrichtung an dem Verbindungsring werden auch die Organengewebe zusammengeführt und gegeneinander mit ihren serösen Außenflächen stößen, wonach durch den Andruck an dem beweglichen Handgriff die Verstellung der Hohlröhre und des zylindrischen Messers sowie die gleichzeitige Bewegung der Nadelvorrichtung zustandekommen, welche mit den Nadeln des anderen Nadelnringes die Gewebe hindurchsticht, und der feststehende Verbindungsring wird in diesem vermittels der Dichtkegel festgelegt. Bei seinem weiteren Verschieben schneidet das zylindrische Messer die Übergewebe und den feststehenden Verbindungsring unter Herausbildung der Anastomosenbohrung ab, durch welche das Ende des Stockes mit der Rundmutter herausgenommen werden kann. Das zwischen der Nadelvorrichtung und dem Verbindungsring vorhandene Gewebe wird zusammengedrückt, wobei die Luftdichtheit der Verbindung, das Zusammenwachsen und die Abstoßung der die Gewebe zusammendrückenden Nadelvorrichtung und des Verbindungsringes ins Innere des röhrenförmigen Organes in 7 bis 10 Tagen erreicht werden, die dann aus dem Organismus auf natürlichem Wege abgeführt werden.

Das betreffende bekannte Nähgerät ist infolge der Verletzung der lebenden Zusammennähungsgewebe im Augenblick der Annäherung des Verbindungsringes und der Nadelvorrichtung und beim Durchstechen nachteilig, wenn das zylindrische Messer die Übergewebe der Zusammennähungswandungen der Organe abschneidet und mit dem Durchschneiden des Verbindungsringes beginnt. In dieser Zeitspanne setzen die Nadeln, obschon sie im Verbindungsring durch die Dichtkegel festeingeklemmt sind, das Eintauchen in diesen Ring dicht bis den Augenblick des endgültigen Durchschneidens des erwähnten Verbindungsringes mit dem Messer fort, wodurch die Überdrückung der Gewebe auftritt.

Da das Durchschneiden des Verbindungsringes mit dem Messer mit dem Vorschieben der Nadeln durch diesen Ring gleichzeitig verläuft, kommt es zur Verformung des Verbindungsringes, die ihrerseits eine Steigerung der Durchstichkraft und demzufolge auch eine zusätzliche Verletzung der lebenden Gewebe verursacht.

Offenbarung der Erfindung

Der vorliegenden Erfindung liegt die Aufgabe zugrunde, ein chirurgisches Nähgerät zu entwickeln, das es ermöglicht, durch eventuelle Abänderung der Zusammenwirkungsart des zylindrischen Messers und der Nadelvorrichtung unter Benutzung nur eines Antriebes den Vorgang des Durchstechens der zusammenzunähenden Gewebe von dem bei dem Ausschneiden der Zentralbohrung in den lebenden Geweben in der Zeit zu trennen.

Die gestellte Aufgabe wird dadurch gelöst, daß das chirurgische Nähgerät zum Anlegen von ringförmigen Kompressionsanastomosen an den Organen des Verdauungskanals, das eine innerhalb des Hohlgehäuses untergebrachte, einen ersteren Bohrungsring mit der Mehrzahl von Bohrungen und dem mit dem ersteren koaxialen anderen Nadelnring mit den an diesem befestigten Nadeln aufweisende Nadelvorrichtung, von welchen Nadeln jede einer Bohrung im ersteren Bohrungsring entgegensteht und mindestens ein Teil an ihrem freien Ende je einen Dichtkegel trägt, eine im Inneren des Hohlgehäuses untergebrachte Stange, an deren einem Ende vermittels Mutter Verbindungsring befestigt ist, durch den bei dem Anlegen der Anastomosen die Nadeln durchstechen, auch die aus diesen Nadeln, die mit den Dichtkegeln ausgestattet sind und im Verbindungsring festeingeklemmt werden, ein hohles zylindrisches Messer, das solchen Innendurchmesser aufweist, daß durch das Messer die Mutter frei durchtritt, und an einer im Hohlgehäuse koaxial untergebrachten Hohlröhre befestigt wird, in die die Stange koaxial eingeht, und ein Antriebswerk des hohlen zylindrischen Messers enthält, das mit der erwähnten Hohlröhre in Wechselwirkung tritt, erfindungsgemäß einen Stößel, der an seinem einen Ende eine Abstützung für den anderen Nadelring der Nadelvorrichtung darstellt und an seinem anderen Ende eine innere Abschrägung und einen Anschlagbund aufweist, der mit einer Anschlagflanke im Hohlgehäuse zwecks Einschränkung der Bewegungsstrecke des anderen Nadelringes bei der Anlegung der Anastomosen zusammenwirkt, eine auf der Hohlröhre aufgesetzte Schlitzbuchse, die Außenkegelbund besitzt, der mit der inneren Abschrägung des Stößels in gegenseitigen Eingriff tritt, und einen Feststeller der Schlitzbuchse aufweist, der auf der Hohlröhre innerhalb der Schlitzbuchse untergebracht ist.

Um eine selbsttätige Bewegung des Stößels zu vermeiden, kann das Nähgerät mit einer Zurückhalterungsvorrichtung ausgestattet werden, die eine Zylinderhülse mit ihrer Ausnehmung, welche Hülse innerhalb des Hohlgehäuses in der Nähe der Hohlröhre untergebracht ist, während auf der Hohlröhre ebenfalls eine Ausnehmung der zylindrischen Gestalt ausgespart ist, die dieser auf der Zylinderhülse entspricht, und einen an dieser Zylinderhülse angelenkten, in Bezug auf das Hohlgehäuse zur Vereinigung der Ausnehmung der Zylinderhülse mit der zylindrischen Ausnehmung der Hohlröhre unter Ermöglichung einer Verstellung der Hohlröhre im Sinne der Vorbereitung des Nähgerätes zum Hindurchstechen verschwenkbaren Handgriff vorsieht.

Zweckmäßigerweise ist der Feststeller der Schlitzbuchse in Form einer weiteren Hohlröhre auszuführen, die mit dem Hohlgehäuse starr gekoppelt und in diesem koaxial untergebracht ist.

Die den Feststeller der Schlitzbuchse darstellende Hohlröhre kann auch die Rolle der Ummantelung des Nähgerätes übernehmen und eine mit der Länge der Stange vergleichbare Streckung aufweisen.

Der Feststeller der Schlitzbuchse kann auch eine mit der Stange koaxiale und mit der an der Durchmesserslinie herum ausgefrästen Bohrung versehene Scheibe und ein Abstützungsstück aufweisen, das innerhalb der erwähnten Diametralbohrung untergebracht ist und seine zum Durchgang der Stange dienende mit der Scheibe koaxiale Bohrung aufweist, während auf der Stange ein Anschlagbund vorgesehen ist, mit dem zwecks Zurückhalterung des Stößels und demzufolge auch der Nadelvorrichtung im Augenblick des Durchschneidens des Verbindungsringes mit dem hohlen zylindrischen Messer das Abstützungsstück in Wechselwirkung tritt.

Es ist nicht minder zweckmäßig, daß die Hohlröhre, an der das hohle zylindrische Messer befestigt ist, die Längsschlitze auf deren Abschnitt der Zusammenwirkung mit der Scheibe des Feststellers der Schlitzbuchse besitzt, in welchen Längsschlitzen das Abstützungsstück in Bezug auf die Hohlröhre bei dem Anlegen der Anastomose verstellt werden kann.

Die Hohlröhre, an der das hohle zylindrische Messer befestigt ist, kann auch eine Ummantelung des Nähgeräts darstellen und eine mit der Länge der Stange vergleichbare Streckung besitzen.

Zweckmäßigerweise ist das Hohlgehäuse außerdem gegen die Hohlröhre, an der das hohle zylindrische Messer befestigt ist, durch eine Feder zu belasten und mit der Scheibe des Feststellers der Schlitzbuchse in Zusammenwirkung unter Ermöglichung zu setzen, den Überdruck an den zusammenzunähenden Geweben bei ihrer erheblichen Stärke herabzusetzen.

Sachgemäß bequem ist noch die Scheibe des Feststellers der Schlitzbuchse gegen die das hohle zylindrische Messer tragende Hohlröhre durch eine weitere Feder zu belasten.

Das erfindungsgemäße chirurgische Nähgerät ermöglicht es, die Verletzungsstärke der zusammenzunähenden Gewebe im Bereich deren Kontaktes bei ihrem Zusammennähen zu vermindern.

Durch Anwendung einer Zurückhalterungsvorrichtung in diesem wird die selbsttätige Bewegung des Stößels während der Vorbereitung des Nähgerätes zum Durchstechen jeweils vermieden.

Durch Benutzung der das hohle zylindrische Messer tragenden Hohlröhre oder der weiteren Hohlröhre des Feststellers als eine Ummantelung wird der Aufbau des ganzen Nähgerätes unter gleichzeitigem Erreichen dessen Gebrauchsbequemlichkeit vereinfacht.

Das federbelastete Hohlgehäuse ermöglicht es, die Verletzbarkeit der Gewebe bei deren erheblicher Stärke zu vermindern.

Durch die Federbelastung der Scheibe des Feststellers wird deren Löslösung vermieden.

## Kurzbeschreibung der Zeichnungen

Nachstehend wird die Erfindung anhand der Beschreibung deren Ausführungsbeispiele unter Bezugnahme auf die begleitenden Zeichnungen näher erläutert, in denen es zeigt:

Fig. 1 erfindungsgemäßes chirurgisches Nähgerät in seinem einen Teil, Längsschnitt;

Fig. 2 teilweise längsgeschnittene Gesamtansicht desselben erfindungsgemäßen chirurgischen Nähgeräts;

Fig. 3 Schnitt III-III aus der Fig. 2;

Fig. 4 Schnitt IV-IV aus der Fig. 1;

Fig. 5 eine andere Ausführungsvariante des erfindungsgemäßen chirurgischen Nähgerätes in längsgeschnittener Gesamtansicht;

Fig. 6 Schnitt VI-VI aus der Fig. 5;

Fig. 7 erfindungsgemäße Schlitzbuchse in Gesamtansicht;

Fig. 8 erfindungsgemäße chirurgische Nähgerät während Durchschneidens des Verbindungsringes im Längsschnitt;

Fig. 9 dasselbe wie in der Fig. 8 für eine andere Ausführungsvariante des erfindungsgemäßen chirurgischen Nähgerätes.

## Beste Ausführungsvariante der Erfindung

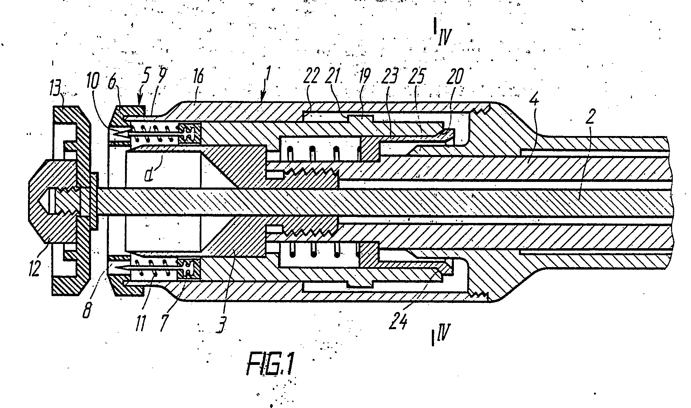

Erfindungsgemäßes chirurgisches Nähgerät zum Anlegen der ringförmigen Kompressionsanastomosen an den Organen des Verdauungskanals enthält ein Hohlgehäuse 1 (Fig. 1), in dessen Innerem eine Stange 2 und das hohle zylindrische Messer 3 koaxial untergebracht sind, welches Messer an einer Hohlröhre 4 befestigt ist. Innerhalb des Hohlgehäuses 1 wird eine die koaxialen Ringe 6 und 7 einschließende abnehmbare Nadelvorrichtung 5 koaxial untergebracht. Im Ring 6 sind Bohrungen 8 ausgespart und im Nadelring 7 die je einer Bohrung 8 entsprechenden Nadeln 9 befestigt. Einige dieser Nadeln 9, nicht weniger als drei aber werden mit je einem Dichtkegel 10 an ihrem Freiende versehen. Der Bohrungsring 6 ist gegen den Nadeln-

ring 7 durch die an einigen Nadeln 9 herum angeordneten Federn 11 belastet.

An einem Ende der Stange 2 ist der Verbindungsring 13 vermittels der Mutter 12 befestigt, der mit der Nadelvorrichtung 5 in Verbindung tritt und die lebenden zusammenzunähenden Gewebe festhält.

Das zylindrische Messer 3 besitzt einen so großen Durchmesser d, daß durch die Innenhöhle des betreffenden Messers 3 die Mutter 12 nach dem Durchschneiden des Verbindungsringes 13 frei hindurchgehen kann.

Die Hohlröhre 4 ist innerhalb des Hohlgehäuses 1 koaxial untergebracht und trägt in ihrem Inneren die Stange 2.

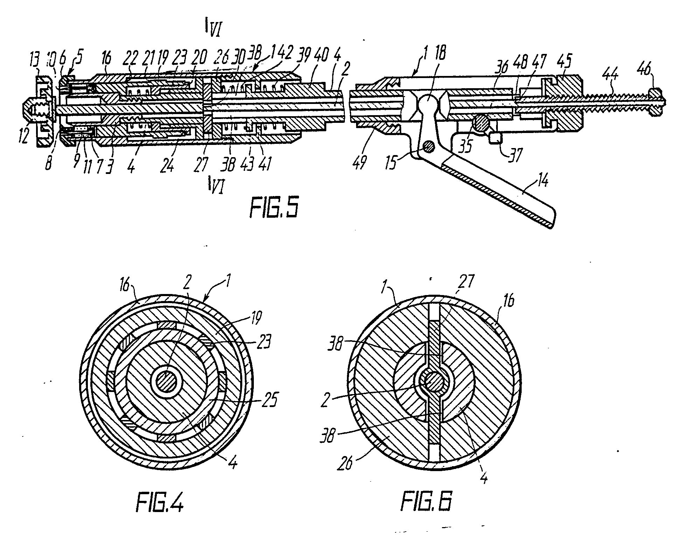

Das Zylindermesser 3 wird weiter mit einem Antrieb in Form des an dem Gelenk 15 gegen das aus drei Einzelteilen 16, 17 (Fig. 3) lösbare Hohlgehäuse 1 verschwenkbaren Handgriffs 14 versehen.

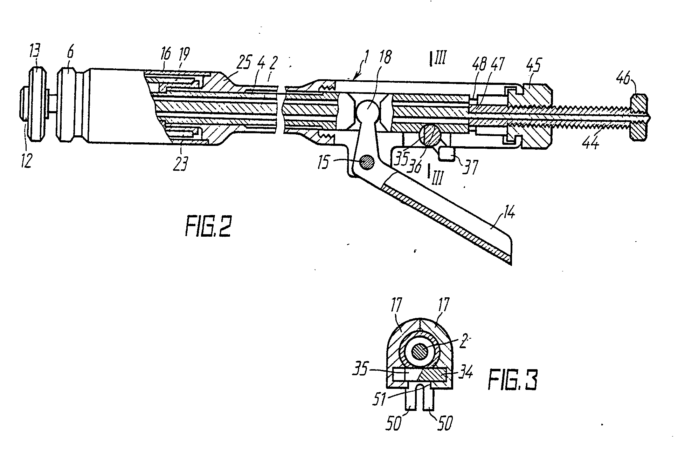

Die Einzelteile 17 des Hohlgehäuses 1 stellen die Einzelteile einer an der Längsachse entlang geschnittenen Hülse dar und bilden an dem Abschnitt der Anordnung des Handgriffs 14 ein feststehendes Gehäuse 1. Der Handgriff 14 (Fig. 2) weist seinen Hebelarm 18 auf, mit dem er mit der Hohlröhre 4 in Wechselwirkung tritt und diese mit dem Zylindermesser 3 zusammenverschiebt.

Der Nadelring 7 (Fig. 1) stößt gegen den Stößel 19 (Fig. 1, 4), der eine innere Abschrägung 20 aufweist. Der Stößel 19 ist mit einer Anschlagsflanke 21 an dem Vorsprungsbund versehen, die mit einer ähnlichen Anschlagsflanke 22 an der Abstufung der inneren Oberfläche des Hohlgehäuses 1 in Wechselwirkung tritt. An der betreffenden Hohlröhre 4 ist eine den mit der inneren Abschrägung 20 des Stößels 19 übereinstimmenden kegeligen Außenbund 24 aufweisende Schlitzbuchse 23 angebracht.

Im Inneren dieser Schlitzbuchse 23 und auch an der Hohlröhre 4 ist ein Feststeller angebracht. In Fig. 1 ist eine Ausführungsvariante des Feststellers in Form von mit dem Hohlgehäuse 1 starrverbundener Hohlröhre 25 wieder-

gegeben. Diese Hohlröhre 25 verläuft dem Hohlgehäuse 1 koaxial und stellt eine Ummantelung des ganzen Nähgeräts dar. Deren Länge ist mit der Länge der Stange 2 vergleichbar und in diesem Zusammenhang umfaßt die Röhre 25 die Hohlröhre 4 mit der Stange 2 in deren Innerem an dem Mittelabschnitt des Nähgerätes. Dabei verbindet die Hohlröhre 25 als die Ummantelung die Gehäuseteile 16 und 17 starr miteinander.

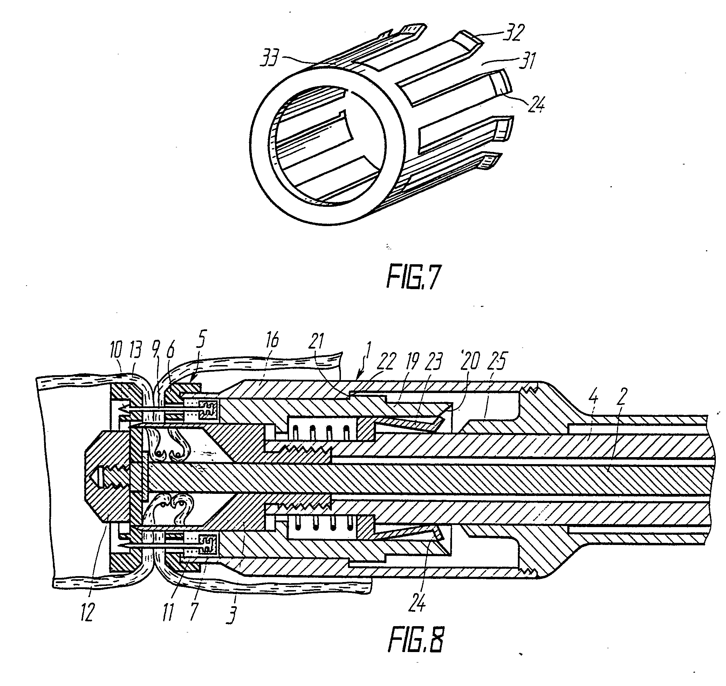

In Fig. 5 ist eine andere Ausführungsvariante des Feststellers der Schlitzbuchse 23 wiedergegeben, der eine im Inneren des Hohlgehäuse 1 mit der Stange 2 koaxial untergebrachte Scheibe 26 und ein Abstützungsstück 27 (Fig. 5, 6) einschließt, das innerhalb einer Diametralbohrung 28 der Scheibe 26 liegt. Zwecks Durchganges der Stange 2 wird in dem Abstützungsstück 27 eine der Scheibe 26 koaxiale Bohrung 29 ausgespart.

An der Strecke der Anordnung des Abstützungsstücke 27 wird auf der Stange 2 eine weitere Anschlagsflanke 30 an deren Abstufung vorgesehen. Als solche Anschlagsflanke 30 kann je auch die Stirnflanke einer jeweiligen Federnut auf der Stange 2 oder diese einer Abflachung auf der Stange 2 hervortreten.

Zwecks Stillhaltens des Stößels 19 und der Nadelvorrichtung 5 während des Durchschneidens des Verbindungsringes 13 mit dem Messer 3 stößt die Anschlagsflanke 30 gegen das Abstützungsstück 27.

Bei dieser Ausführungsvariante des Feststellers der Schlitzbuchse 23 mit der Scheibe 26 dient die Hohlröhre 4 (Fig. 5) als die Ummantelung des Nähgeräts. Dabei ist die Länge der erwähnten Hohlröhre 4 mit dieser der Stange 2 vergleichbar.

Die Schlitzbuchse 23 (Fig. 7) stellt einen hohlen Zylinder dar, dessen Mantelfläche durch die durchgehenden Schlitze 31 in vermittels des nichtgeschnittenen Abschnitts 33 des Zylinders selbst vereinten Lamellen 32 eingeteilt wird. An Freienden jeder Lamelle 32 ist je ein Kegelbund 24 vorgesehen. Die Anzahl der Lamellen 32 bestimmt die für das Durchstechen des Verbindungsringes 13 mit den Nadeln 9 erforderliche Kraft, d.h. die Durchstichkraft. Das Nähgerät ist noch mit einer der selbsttätigen Bewegung des Stößels 19 vorbeugenden Zurückhalterungsvorrichtung versehen, die eine zwischen den Einzelteilen 17 des Hohlgehäuses 1 der Achse der Stange senkrecht verlegte Zylinderklinke 34 (Fig. 2 bzw. 3 und 5) einschließt. An dieser Zylinderklinke 34 ist eine der am Abschnitt der Hohlröhre 4 in der Nähe der Anordnung der betreffenden Zurückhalterungsvorrichtung ausgefrästen zylindrischen Ausnehmung 36 entsprechende Ausnehmung 35 ausgespart. An der Zylinderklinke 34 ist noch ein die erwähnte Klinke 34 bis zum Zusammenfallen beider Ausnehmungen 35 und 36 zwecks Löslösung der Hohlröhre 4 zu deren Verstellung drehender Hebel 37 befestigt.

An deren Abschnitt der Zusammenwirkung mit der Scheibe 26 wird die Hohlröhre 4 (Fig. 5) mit Längseinschnitten 38 versehen, in denen sich das Abstützungsstück 27 bezüglich der Röhre 4 bei dem Anlegen der Anastomosen hin- und herbewegen kann.

Das Hohlgehäuse 1 ist durch die Feder 39 gegen die Hohlröhre 4 belastet, welche Feder auf der Hohlröhre 4 zwischen deren Vorsprung 40 und dem am Hohlgehäuse 1 vorgesehenen Vorsprung 41 aufgesetzt ist.

Die Scheibe 26 des Feststellers der Schlitzbuchse 23 ist durch die Feder 42 gegen die Hohlröhre 4 belastet, die auf der erwähnten Hohlröhre 4 zwischen deren Vorsprung 43 und der Stirnfläche der Scheibe 26 angebracht ist.

Das hohle zylindrische Messer 3 ist auf der Hohlröhre 4 vermittels Gewindeverbindung befestigt.

Die Stange 2 geht im Inneren einer mit der in den Einzelteilen 17 des Hohlgehäuses 1 unter Ermöglichung deren Drehung eingebauten Mutter 45 verbundenen Hohlschraube 44 hindurch. An dem freien Rückendeteil der Stange 2 ist das Gewinde ausgefräst und eine Mutter 46 aufgeschraubt. Durch Drehung an der Mutter 46 wird die Stange 2 aus der Hohlschraube 44 bis zum Anschlagen an das Stirnende der Schraube 44 mit deren Stirnfläche 47 herausgezogen, wobei die Hohlschraube 44 infolge des nichtrunden Einschnitts 48 in den Einzelteilen 17 des Hohlgehäuses 1 nicht gedreht werden kann.

Die Einzelteile 17 des Hohlgehäuses 1 werden durch die Mutter 49 zusammengekoppelt, in deren Innerem die Hohlröhre 4 hindurchgezogen wird.

Der Hebel 37 der Zurückhalterungsvorrichtung besteht aus zwei federnden, in den Ausschnitt 51 in den Einzelteilen 17 des Hohlgehäuses 1 hineinragenden Teilhebeln 50 (Fig. 3).

Das erfindungsgemäße chirurgische Nähgerät arbeitet wie folgt.

Das erfindungsgemäße chirurgische Nähgerät ist zum Anlegen von ringförmigen Kompressionsanastomosen an Hohlorganen des Verdauungskanals, und zwar der End-an--End, End-an-Seite- und Seite-an-Seite-Anastomosen vorausbestimmt. Nachstehend wird ein Beispiel der Anlegung einer End-an-End-Anastomose bei Vorderresektion des Mastdarms beschrieben.

Nach der Durchführung der erwähnten Resektion werden die Tabaksbeutelnähte an dem Stumpf des Mastdarms und an dem Ende des S-ähnlichen Dickdarmabschnitts angelegt. Über die Analöffnung wird in den Mastdarm das Nähgerät eingeführt, wobei der Verbindungsring 13 und die Nadelvorrichtung 5 angenähert sind. Hiernach wird das Vorderende der Stange 2 mit dem an diesem befestigten Verbindungsring 13 aus dem Bereich des Mastdarms vorgeschoben und die Tabaksbeutelnaht an dem Mastdarmstumpf zusammengezogen. Im weiteren wird das Ende des S-ähnlichen Dickdarms auf das Vorderende der Stange 2 mit dem Verbindungsring 13 daran aufgeschoben und die Tabaksbeutelnaht an dem S-ähnlichen Dickdarm ebenfalls festeingezogen. Nach dem Abschneiden der Überfaden der Tabaksbeutelnähte wird weitere Annäherung des Verbindungsringes 13 und der Nadelvorrichtung 5 aneinander bis auf die Durchstichstellung durch Drehung an der Mutter 45 durchgeführt. Hiernach wird die Zylinderklinke 34 ver-

mittels des Hebels 37 der Zurückhalterungsvorrichtung bis zum Zusammenfallen der Ausnehmungen 35 und 36 gedreht, d.h. die Hohlröhre 4 wird freigegeben. Dann wird es am Handgriff 14 gedrückt, der sich an dem Gelenk 15 verschwenkt. In diesem Zusammenhang schiebt der Hebelarm 18 unter dessen Zusammenwirkung mit der Hohlröhre 4 diese mit dem zylindrischen Messer 3 und an ihr befestigten Schlitzbuchse 23 zusammen vorwärts, in deren Innerem der Feststeller in Form von hohler Röhre 25 untergebracht ist.

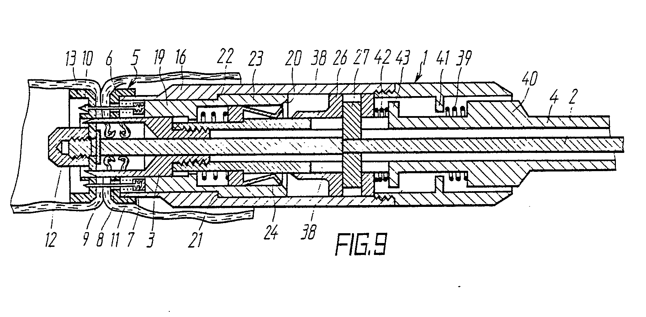

Die Schlitzbuchse 23 greift mit ihrem kegeligen Außenbund 24 in die innere Abschrägung 20 des Stößels 19 ein und verschiebt ihn mit dem Nadelring 7 der Nadelvorrichtung 5 zusammen wiederum vorwärts. Die erwähnten Nadeln 9 stechen die Gewebe und den Verbindungsring 13 unter deren Durchgang durch die Bohrungen 8 im Bohrungsring 6 und Festeinklemmung in diesem Verbindungsring 13 vermittels der Nadeln 9 mit den Dichtkegeln 10 hindurch. Zu diesem Augenblick befindet sich der Feststeller 25, der mit dem Hohlgehäuse 1 starrverbunden ist, im Innenraum der Schlitzbuchse 23, während der Stößel 19 nach seinem Eingriff in die Anschlagsflanke 22 des Gehäuses 1 mit seiner Anschlagsflanke 21 stillgelegt wird, ohne jeweiligen Druck an dem Nadelring 7 der Nadelvorrichtung 5 auszuüben. Bei der weiteren Vorschubbewegung der Hohlröhre 4 tritt die Schlitzbuchse 23 in den Innenraum des Stößels 19 (Fig. 8) hinein, ohne diesen zu verstellen, während das zylindrische Messer 3 die Übergewebe ab- und eine Bohrung in dem Verbindungsring 13 ausschneidet.

Bei einer anderen Ausführungsvariante des Feststellers der Schlitzbuchse 23 (Fig. 5) greift die Stange 2 mit ihrer Anschlagsflanke 30 bei der Vorschubbewegung der Hohlröhre 4 in das Abstützungsstück 27 ein, das in der Diametralbohrung der Scheibe 26 untergebracht ist und in die Längsausschnitte 38 der Hohlröhre 4 hineinragt, während die unbeweglich bleibende Scheibe 26 aus dem Innenraum der Schlitzbuchse 23 heraustritt, indem sie diese Buchse im nächsten Augenblick nach dem Durchstechen des Verbindungsringes 13 mit den Nadeln 9 und nach der Festeinklemmung der Nadeln 9 in diesem Ring durch die Dichtkegel 10 freigibt.

Bei der weiteren Vorschubbewegung der Hohlröhre 4 tritt die Schlitzbuchse 23 in den Innenraum des Stößels 19 ohne dessen Verstellung hinein, während das zylindrische Messer 3 die Übergewebe ab- und eine Bohrung im Verbindungsring 13 (Fig. 9) ausschneidet. Jetzt kann das Nähgerät aus dem Mastdarm herausgenommen werden, während die Nadelvorrichtung 5 mit dem Verbindungsring 13 zusammen in der Höhle des Darms zurückbleibt und nach außen in 5 bis 7 Tagen nach der durchgeführten Operation mit den zwischen der Nadelvorrichtung 5 und dem Verbindungsring 13 zusammengedrückten nekrotisierten Geweben auf natürlichem Wege herausgestoßen wird. Zu dieser Zeit erfolgt auch das Zusammenwachsen der Darmwandungen an dem Außenumfang bezüglich der Zusammendrückungszone. Bis die Nadelvorrichtung 5 mit dem Verbindungsring 13 zusammen abgestoßen wird, kann der Eingeweideninhalt durch die mit dem zylindrischen Messer 3 herausgeschnittene Bohrung hindurchtreten.

Mit Hilfe des erfindungsgemäßen chirurgischen Nähgeräts wurden 12 Operationen im Forschungslabor an den Hunden und 20 Operationen am Magen, an Dünn- bzw. Dickdarm durchgeführt.

Bei Tieren ist eine gutgeformte Anastomose ohne Narbenverdickung vermerkt worden.

Bei den Operationen am Magen wurden bei den Kranken die Verbindungsringe des jeweiligen Durchmessers ausgenutzt, die in allen Fällen auf natürlichem Wege frei ausgesondert waren. Es sind auch keine Komplikationen nach den durchgeführten Operationen nachgewiesen. Hinsichtlich der vorhandenen Verkleinerung der Verletzungsstärke der lebenden Zusammennähungsgewebe heilten die operierten Kranken um 5 bis 7 Tage früher als die Kontrollkranken aus.

## Industrielle Anwendbarkeit

Die Erfindung kann bei den Operationen von Organen des Verdauungskanals, bevorzugt von Intestinum, Magen und Speiseröhre ihre Anwendung finden.

0324858

## PATENTANSPRÜCHE

1. Chirurgisches Nähgerät zum Anlegen von ringförmigen Kompressionsanastomosen an den Organen des Verdauungskanals, das eine im Inneren des Hohlgehäuses (1) untergebrachte Nadelvorrichtung (5), die einen ersteren Bohrungsring (6) mit einer Mehrzahl der Bohrungen (8) drin und einen mit diesem koaxialen anderen Nadelnring (7) mit den an diesem befestigten Nadeln (9) aufweist, jeder von denen je eine Bohrung (8) des ersteren Bohrungsringes (6) entspricht und von denen mindestens einige Nadeln an ihrem freien Ende je einen Dichtkegel (10) tragen, eine innerhalb des Hohlgehäuses (1) verlegte Stange (2), an deren Vorderende ein Verbindungsring (13) vermittels der Mutter (12) unbeweglich befestigt ist, durch den während des Anlegens der Anastomosen die Nadeln (9) durchstechen und in dem die den Dichtkegel (10) tragenden Nadeln im Verbindungsring (13) festeingeklemmt werden, ein hohles zylindrisches, an einer im Inneren des Hohlgehäuses (1) koaxial untergebrachten Hohlröhre (4), in der die Stange koaxial verlegt ist, befestigtes Messer (3), das einen solchen inneren Durchmesser besitzt, daß durch das Messer die erwähnte Mutter (12) frei hindurchtritt, und ein Antriebswerk des hohlen zylindrischen Messers (3) vorsieht, das mit der Hohlröhre (4) zusammenwirkt, d a d u r c h   g e - k e n n z e i c h n e t, daß es einen Stößel (19), an dessen einem Ende der andere Nadelring (7) der Nadelvorrichtung (5) stößt und an dem anderen Ende eine innere Abschrägung (20) und eine mit der Anschlagsflanke (22) in dem Hohlgehäuse (1) zwecks Einschränkung des Bewegungsweges des anderen Nadelrings (7) bei dem Anlegen der Anastomosen in Wechselwirkung tretende Anschlagsflanke (21) ausgeführt sind, eine auf der Hohlröhre (4) aufgesetzte, mit einem kegeligen, mit der inneren Abschrägung (20) des Stößels (19) in Eingriff tretenden Außenbund (24) versehene Schlitzbuchse (23) und Feststeller der Schlitzbuchse (23) einschließt, der auf der Hohlröhre (4)

0324858

innerhalb der Schlitzbuchse (23) angeordnet ist.

2. Chirurgisches Nähgerät nach Anspruch 1, d a - d u r c h   g e k e n n z e i c h n e t , daß zwecks Vermeidung einer selbsttätigen Bewegung des Stößels (19) dieser eine Zurückhalterungsvorrichtung, die eine Zylinderklinke (34) mit ihrer Ausnehmung (35), welche Klinke im Inneren des Hohlgehäuses (1) in der Nähe der Hohlröhre (4) untergeordnet ist, an der auch eine zylindrische, der Zylinderklinke (34) entsprechende Ausnehmung (36) ausgespart ist, und einen an der erwähnten Zylinderklinke (34) befestigten, zum Vereinigen der Ausnehmung (35) auf der Zylinderklinke (34) mit der zylindrischen Ausnehmung (36) der Hohlröhre (4) in bezug auf das Hohlgehäuse (1) verschwenkbaren Hebel (37) aufweist, indem das Nähgerät zum Durchstechen dadurch vorbereitet und die Verstellung der Hohlröhre (4) ermöglicht wird.

3. Chirurgisches Nähgerät nach Anspruch 1 bzw. 2, d a d u r c h   g e k e n n z e i c h n e t , daß der Feststeller der Schlitzbuchse (23) als eine Hohlröhre (25) ausgeführt ist, die mit dem Hohlgehäuse (1) starr verbunden und diesem koaxial angeordnet ist.

4. Chirurgisches Nähgerät nach Anspruch 3, d a - d u r c h   g e k e n n z e i c h n e t , daß die den Feststeller der Schlitzbuchse (23) darstellende Hohlröhre (25) als eine Ummantelung für das chirurgische Nähgerät dient und eine mit der Länge der Stange (2) vergleichbare Streckung aufweist.

5. Chirurgisches Nähgerät nach Anspruch 1 bzw. 2, d a d u r c h   g e k e n n z e i c h n e t , daß der Feststeller der Schlitzbuchse (23) eine mit der Stange (2) koaxiale, mit einer an der Durchmesserslinie herum verlegte Diametralbohrung (28) versehene Scheibe (26) und ein in dieser Diametralbohrung (28) der Scheibe (26) montiertes Abstützungsstück (27) enthält, das seine zwecks Durchganges der Stange (2) mit der Scheibe (26) koaxiale Eigenbohrung besitzt, während auf der Stange (2) eine Anschlagsflanke (30) vorgesehen ist, die mit dem Abstützungsstück (27) zum Stillegen des Stößels (19) und
demzufolge auch der Nadelvorrichtung (5) im Augenblick
des Durchschneidens des Verbindungsringes (13) mit dem
hohlen, zylindrischen Messer (3) in Wechselwirkung
tritt.

6. Chirurgisches Nähgerät nach Anspruch 5, d a -
d u r c h   g e k e n n z e i c h n e t , daß die Hohlröhre (4), an der das hohle, zylindrische Messer (3) befestigt ist, an deren Abschnitt der Zusammenwirkung mit
der Scheibe (26) des Feststellers der Schlitzbuchse (23)
mit Längseinschnitten (38) versehen ist, in denen sich
das Abstützungsstück (27) bezüglich der Hohlröhre (4)
bei dem Anlegen der Anastomosen hin- und herbewegen kann.

7. Chirurgisches Nähgerät nach Anspruch 5, d a -
d u r c h   g e k e n n z e i c h n e t , daß die Hohlröhre (4), an der das hohle, zylindrische Messer (3) befestigt ist, eine Ummantelung des Nähgeräts darstellt
und eine mit der Länge der Stange (2) vergleichbare
Streckung aufweist.

8. Chirurgisches Nähgerät nach Anspruch 6, d a -
d u r c h   g e k e n n z e i c h n e t , daß die Hohlröhre (4), an der das hohle zylindrische Messer (3) befestigt ist, eine Ummantelung des Nähgeräts darstellt
und eine mit der Länge der Stange (2) vergleichbare
Streckung aufweist.

9. Chirurgisches Nähgerät nach Anspruch 5, d a -
d u r c h   g e k e n n z e i c h n e t , daß das Hohlgehäuse (1) gegen die Hohlröhre (4), an der das hohle zylindrische Messer (3) befestigt ist, durch eine Feder
belastet ist und mit der Scheibe (26) des Feststellers
der Schlitzbuchse (23) in Wechselwirkung tritt, indem
dadurch der Überdruck an den zusammenzunähenden Geweben
bei ihrer erheblichen Stärke herabgesetzt werden.

10. Chirurgisches Nähgerät nach Anspruch 6, d a -
d u r c h   g e k e n n z e i c h n e t , daß das Hohlgehäuse (1) gegen die Hohlröhre (4), an der das hohle

zylindrische Messer (3) befestigt ist, durch eine Feder belastet ist und mit der Scheibe (26) des Feststellers der Schlitzbuchse (23) in Wechselwirkung tritt, indem dadurch der Überdruck an den zusammenzunähenden Geweben bei ihrer erheblichen Stärke herabgesetzt werden kann.

11. Chirurgisches Nähgerät nach Anspruch 7, d a d u r c h   g e k e n n z e i c h n e t, daß das Hohlgehäuse (1) gegen die Hohlröhre (4) an der das hohle zylindrische Messer (3) befestigt ist, durch eine Feder belastet ist und mit der Scheibe (26) des Feststellers der Schlitzbuchse (23) in Wechselwirkung tritt, indem dadurch der Überdruck an den zusammenzunähenden Geweben bei ihrer erheblichen Stärke herabgesetzt werden. kann.

12. Chirurgisches Nähgerät nach jedem der Ansprüche 5 bis 8, d a d u r c h   g e k e n n z e i c h n e t, daß die Scheibe (26) des Feststellers der Schlitzbuchse (23) gegen die Hohlröhre (4), an der das hohle zylindrische Messer (3) befestigt ist, federbelastet ist.

13. Chirurgisches Nähgerät nach Anspruch 9, d a d u r c h   g e k e n n z e i c h n e t, daß die Scheibe (26) des Feststellers der Schlitzbuchse (23) gegen die Hohlröhre (4), an der das hohle zylindrische Messer (3) befestigt ist, federbelastet ist.

FIG.1

**FIG.2**

**FIG.3**

FIG.5

FIG.4

FIG.6

4/5

FIG.7

FIG.8

5/5

FIG.9

# INTERNATIONAL SEARCH REPORT

International Application No PCT/SU 88/00020

---

## I. CLASSIFICATION OF SUBJECT MATTER (if several classification symbols apply, indicate all) ⁵

According to International Patent Classification (IPC) or to both National Classification and IPC

IPC⁴  A 61 B 17/11

---

## II. FIELDS SEARCHED

### Minimum Documentation Searched ⁷

| Classification System | Classification Symbols |
|---|---|
| IPC⁴ | A 61 B 17/04, 17/08, 17/10, 17/11 |

### Documentation Searched other than Minimum Documentation to the Extent that such Documents are Included in the Fields Searched ⁸

---

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| A | US, A, 4476863 (Nikolai N. Kenshin et al.) 16 October 1984 (16.10.84), see the abstract | 1 |
| A | SU, A1, 1225541 (Vsesojuzny nauchnoris-sledovatelsky i ispytatelny institut meditsinskoi tekhniki) 23 April 1986 (23.04.86) see the abstract | 2 |
| A | SU, A1, 1235495 (Vsesojuzny nauchnoris-sledovatelsky i ispytatelny institut meditsinskoi tekhniki et al.) 7 June 1986 (07.06.86), see the abstract | 3 |
| A | US, A, 4573468 (United States Surgical Corporation) 4 March 1986 (04.03.86) see the abstract | 4,5,7,8 |
| A | US, A, 4552148 (Americain Cyanamid Company) 12 November 1985 (12.11.85) see the abstract ./. | 6 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

---

## IV. CERTIFICATION

| Date of the Actual Completion of the International Search | Date of Mailing of this International Search Report |
|---|---|
| 4 April 1988 (04.07.88) | 8 August 1988 (08.08.88) |

| International Searching Authority | Signature of Authorized Officer |
|---|---|
| ISA/SU | |

Form PCT/ISA/210 (second sheet) (January 1985)

0324858

| FURTHER INFORMATION CONTINUED FROM THE SECOND SHEET | |
|---|---|
| A    SU, A1, 1286182 (Krymsky gosudarstvenny meditsinsky institut) 30 January 1987 (30.01.87) see the abstract<br><br>----------------- | 9-13 |

**V.☐ OBSERVATIONS WHERE CERTAIN CLAIMS WERE FOUND UNSEARCHABLE ¹**

This international search report has not been established in respect of certain claims under Article 17(2) (a) for the following reasons:

1.☐ Claim numbers............, because they relate to subject matter not required to be searched by this Authority, namely:

2.☐ Claim numbers............, because they relate to parts of the international application that do not comply with the prescribed requirements to such an extent that no meaningful international search can be carried out, specifically:

3.☐ Claim numbers............, because they are dependent claims and are not drafted in accordance with the second and third sentences of PCT Rule 6.4(a).

**VI.☐ OBSERVATIONS WHERE UNITY OF INVENTION IS LACKING ²**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Mark on Protest

☐ The additional search fees were accompanied by applicant's protest.

☐ Protest accompanied the payment of additional search fees.